# EUROPÄISCHE PATENTSCHRIFT

(11) **EP 1 037 599 B1**
(45) Veröffentlichungstag und Bekanntmachung des Hinweises auf die Patenterteilung: **06.10.2004**
(21) Anmeldenummer: 98964459.6
(22) Anmeldetag: 28.11.1998
(51) Int. Cl.: A61K 7/50

(54) **VERWENDUNG VON DETERGENSGEMISCHEN ENTHALTEND ESTERQUATS UND ALKOXYLIERTE FEESÄUREAMIDOAMINE ZUR HERSTELLUNG VON KOSMETISCHEN ZUBEREITUNGEN**
UTILIZATION OF DETERGENT MIXTURES
UTILISATION DE MELANGES DETERGENTS

(30) Priorität: 08.12.1997 DE 19754283
(43) Veröffentlichungstag der Anmeldung: 27.09.2000
(73) Patentinhaber: Cognis Deutschland GmbH & Co. KG, 40589 Düsseldorf (DE)
(72) Erfinder: BIGORRA, Joaquin, E-08203 Sabadell (ES); PI SUBIRANA, Rafael, E-08400 Granollers (ES); PRAT QUERALT, Esther, E-08328 Alella (ES)
(86) Internationale Anmeldenummer: PCT/EP1998/007699
(87) Internationale Veröffentlichungsnummer: WO 1999/029283

(56) Entgegenhaltungen:
- EP-A- 0 465 734
- DE-A- 3 926 740
- DE-C- 4 308 794
- US-A- 4 837 013
- CHEMICAL ABSTRACTS, vol. 121, no. 2, 1994 Columbus, Ohio, US; abstract no. 17706z, Z. VODAK ET AL : "Preparation of hair conditioners" Seite 575; XP002104680 & CS 276 330 A13. Mai 1992
- DATABASE WPI Week 9216 Derwent Publications Ltd., London, GB; AN 92-126449 XP002104753 "Hair rinse compsn. for hair conditioning - contains lanolin quat. ammonium salt and tert. amide amine" & JP 04 066520 A (KANEBO), 2. März 1992
- DATABASE WPI Week 9705 Derwent Publications Ltd., London, GB; AN 97-048233 XP002104681 "Skin care toiletry - has anionic surfactant contg. peptide gp. and each 2 hydrophobic and hydrophilic gps." & JP 08 301747 A (KANEBO) , 19. November 1996
- DATABASE WPI Week 9728 Derwent Publications Ltd., London, GB; AN 97-306539 XP002104682 "Hair treating agent having good feel and biodegradation property - comprises organic acid of quat. ammonium and neutralisation product from organic acid and amine" & JP 09 118606 A (SANYO CHEM IND) , 6. Mai 1997

## Beschreibung

### Gebiet der Erfindung

Die Erfindung betrifft die Verwendung von Detergensgemischen, enthaltend Esterquats und alkoxylierte Fettsäureamidoamine zur Herstellung von kosmetischen Zubereitungen.

### Stand der Technik

Im Zuge gestiegener Anforderungen an die biologische Abbaubarkeit haben quartäre Fettsäurealkoanolimestersalze, die sogenannten "Esterquats", Tetraalkylammoniumverbindungen als kationische Tenside sowohl für die Herstellung Wäscheweichspülmitteln als auch für die Haaravivage verdrängt. Übersichten zu diesem Thema sind beispielsweise von R.Puchta et al. in **Tens.Surf.Det., 30, 186 (1993),** M.Brock in **Tens.Surf.Det. 30, 394 (1993),** R.Lagerman et al. in **J.Am.Oil.Chem.Soc., 71, 97 (1994)** sowie I.Shapiro in **Cosm.Toil. 109, 77 (1994)** erschienen. Im Markt besteht jedoch der Wunsch nach Produkten mit weiter verbesserten Eigenschaften.

In diesem Zusammenhang sei auf die Deutsche Patentschrift **DE-C2 3926740** (REWO) verwiesen, in der Textilweichspülmittel vorgeschlagen werden, die Esterquats und alkoxylierte Fettsäureamidoamine enthalten.

Die komplexe Aufgabe der Erfindung hat somit darin bestanden, Detergensgemische zur Verfügung zu stellen, die über verbesserte avivierende und antistatische Eigenschaften sowie vollständige biologische Abbaubarkeit verfügen, eine ausreichend hohe Viskosität besitzen, jedoch bei Lagerung nicht eindicken und Gele bilden, und schließlich ohne Mitverwendung von Alkoholen in beliebigem Verhältnis mit Wasser leicht löslich sind.

### Beschreibung der Erfindung

Gegenstand der Erfindung ist die Verwendung von Detergensgemischen, enthaltend
(a) Esterquats, erhältlich durch Kondensation von Fettsäuren mit Alkanolaminen und nachfolgende Quatemierung der Alkanolaminoligoester, und
(b) alkoxylierte Fettsäureamidoamine der Formel **(IV)** einsetzt, 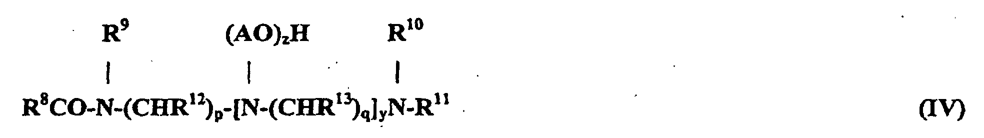 in R⁸CO für einen aliphatischen, linearen oder verzweigten, gesättigten oder ungesättigten Acylrest mit 6 bis 22 Kohlenstoffatomen, R⁹ und R¹⁰ unabhängig voneinander für Wasserstoff oder einen gegebenenfalls hydroxysubstituierten Alkylrest mit 1 bis 4 Kohlenstoffatomen, R¹¹ für Wasserstoff, einen Alkylrest mit 1 bis 4 Kohlenstoffatomen oder R⁸CO, R¹² und R¹³ unabhängig voneinander für Wasserstoff oder eine Methylgruppe, A für eine lineare oder verzweigte Alkylen-gruppe mit 2 bis 4 Kohlenstoffatomen, p und q unabhängig voneinander für Zahlen von 1 bis 3, y für Zahlen von 1 bis 3 und z für Zahlen von 1 bis 20 steht,
zur Herstellung von kosmetischen Zubereitungen, insbesondere Haarpflege- und Haarkonditioniermitteln.

Überraschenderweise wurde gefunden, daß die erfindungsgemäß zu verwendenden Gemische eine vorteilhaft hohe und stabile Viskosität aufweisen. Die Zubereitungen lassen sich in beliebigen Mengen mit Wasser verdünnen und können dann sofort als Haarpflege- oder Konditioniermittel eingesetzt werden. Keratinfasern verleihen sie einen besonders angenehmen Weichgriff, zudem reduzieren sie die statische Aufladung zwischen den Faserfilamenten ganz erheblich. Es empfiehlt sich, den Mischungen zur Einstellung einer höheren Viskosität Fettalkohole oder Emulgatoren zuzusezen.

### Esterquats

Unter der Bezeichnung "Esterquats" werden im allgemeinen quatemierte Fettsäuretriethanolaminestersalze verstanden. Es handelt sich dabei um bekannte Stoffe, die man nach den einschlägigen Methoden der präparativen organischen Chemie erhalten kann. In diesem Zusammenhang sei auf die Internationale Patentanmeldung **WO 91/01295** (Henkel) verwiesen, nach der man Triethanolamin in Gegenwart von unterphosphoriger Säure mit Fettsäuren partiell verestert, Luft durchleitet und anschließend mit Dimethylsulfat oder Ethylenoxid quaterniert. Aus der Deutschen Patentschrift **DE-C1 4308794** (Henkel) ist überdies ein Verfahren zur Herstellung fester Esterquats bekannt, bei dem man die Quaternierung von Triethanolaminestern in Gegenwart von geeigneten Dispergatoren, vorzugsweise Fettalkoholen, durchführt. Die quaternierten Fettsäuretriethanolaminestersalze folgen beispielsweise der Formel (I), 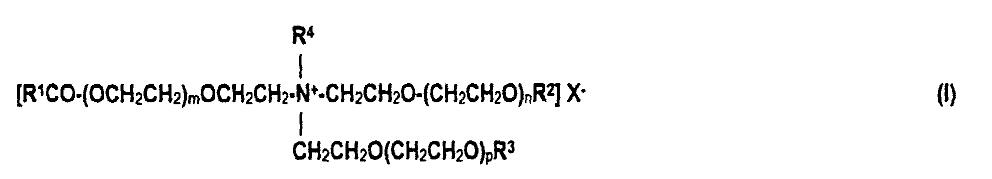 in der R¹CO für einen Acylrest mit 6 bis 22 Kohlenstoffatomen, R² und R³ unabhängig voneinander für Wasserstoff oder R¹CO, R⁴ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht. Typische Beispiele für Esterquats, die im Sinne der Erfindung Verwendung finden können, sind Produkte auf Basis von Capronsäure, Caprylsäure, Caprinsäure, Laurinsäure, Myristinsäure, Palmitinsäure, Isostearinsäure, Stearinsäure, Ölsäure, Elaidinsäure, Arachinsäure, Behensäure und Erucasäure sowie deren technische Mischungen, wie sie beispielsweise bei der Druckspaltung natürlicher Fette und Öle anfallen. Vorzugsweise werden technische C_{12/18}-Kokosfettsäuren und insbesondere teilgehärtete C_{16/18}-Talg- bzw. Palmfettsäuren sowie elaidinsäurereiche C_{16/18}-Fettsäureschnitte eingesetzt. Zur Herstellung der quaternierten Ester können die Fettsäuren und das Triethanolamin im molaren Verhältnis von 1,1 : 1 bis 3 : 1 eingesetzt werden. Im Hinblick auf die anwendungstechnischen Eigenschaften der Esterquats hat sich ein Einsatzverhältnis von 1,2 : 1 bis 2,2 : 1, vorzugsweise 1,5 : 1 bis 1,9 : 1 als besonders vorteilhaft erwiesen. Die bevorzugten Esterquats stellen technische Mischungen von Mono-, Di- und Triestem mit einem durchschnittlichen Veresterungsgrad von 1,5 bis 1,9 dar und leiten sich von technischer C_{16/18}- Talg- bzw. Palmfettsäure (lodzahl 0 bis 40) ab. Aus anwendungstechnischer Sicht haben sich quatemierte Fettsäuretriethanolaminestersalze der Formel (I) als besonders vorteilhaft erwiesen, in der R¹CO für einen Acylrest mit 16 bis 18 Kohlenstoffatomen, R² für R¹CO, R³ für Wasserstoff, R⁴ für eine Methylgruppe, m, n und p für 0 und X für Methylsulfat steht.

Neben den quaternierten Fettsäuretriethanolaminestersalzen kommen als Esterquats ferner auch quaternierte Estersalze von Fettsäuren mit Diethanolalkylaminen der Formel (II) in Betracht, 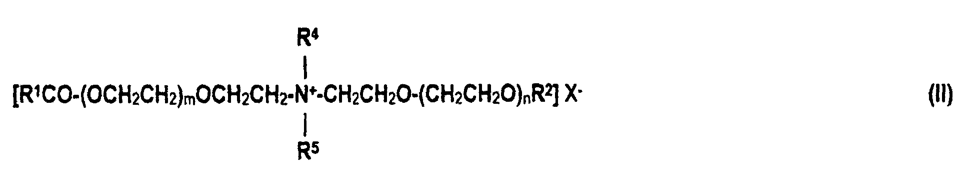 in der R¹CO für einen Acylrest mit 6 bis 22 Kohlenstoffatomen, R² für Wasserstoff oder R¹CO, R⁴ und R⁵ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

Als weitere Gruppe geeigneter Esterquats sind schließlich die quaternierten Estersalze von Fettsäuren mit 1,2-Dihydroxypropyldialkylaminen der Formel (III) zu nennen, 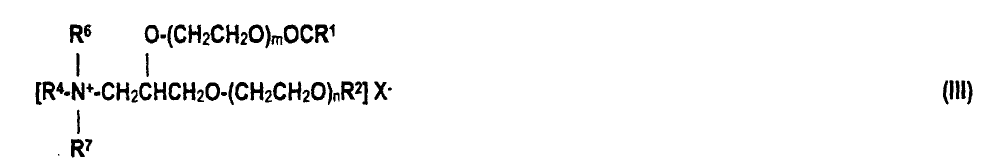 in der R¹CO für einen Acylrest mit 6 bis 22 Kohlenstoffatomen, R² für Wasserstoff oder R¹CO, R⁴, R⁶ und R⁷ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

Hinsichtlich der Auswahl der bevorzugten Fettsäuren und des optimalen Veresterungsgrades gelten die für (I) genannten Beispiele auch für die Esterquats der Formeln (II) und (III). Üblicherweise gelangen die Esterquats in Form 50 bis 90 Gew.-%iger alkoholischer Lösungen in den Handel, die bei Bedarf problemlos mit Wasser verdünnt werden können.

### Alkoxylierte Fettsäureamidoamine

Alkoxylierungsprodukte von Fettsäureamidoaminen sind ebenfalls literaturbekannt und folgen der Formel **(II)**, 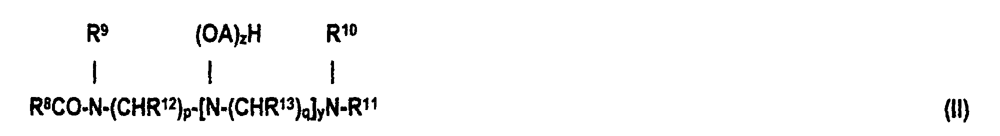 in R⁸CO für einen aliphatischen, linearen oder verzweigten, gesättigten oder ungesättigten Acylrest mit 6 bis 22 Kohlenstoffatomen, R⁹ und R¹⁰ unabhängig voneinander für Wasserstoff oder einen gegebenenfalls hydroxysubstituierten Alkylrest mit 1 bis 4 Kohlenstoffatomen, R¹¹ für Wasserstoff, einen Alkylrest mit 1 bis 4 Kohlenstoffatomen oder R⁸CO, R¹² und R¹³ unabhängig voneinander für Wasserstoff oder eine Methylgruppe, A für eine lineare oder verzweigte Alkylengruppe mit 2 bis 4 Kohlenstoffatomen, p und q unabhängig voneinander für Zahlen von 1 bis 3, y für Zahlen von 1 bis 3 und z für Zahlen von 1 bis 20 steht.

Zu ihrer Herstellung geht man üblicherweise von Dialkylentriaminen oder Trialkylentetraminen aus, die zunächst mit 1 bis 2 Mol Carbonsäure verestert und dann in an sich bekannter Weise unter Insertion in die freien NH-Bindungen mit Alkylenoxiden, vorzugsweise Ethylenoxid umgesetzt werden. Typische Beispiele für geeignete Oligoamine sind Diethylentriamin, Dipropylentriamin, Triethylentetramin und Tripropylentetramin sowie deren Gemische. Als Carbonsäuren kommen die Fettsäuren mit 6 bis 22 Kohlenstoffatomen in Frage, wie z.B. Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Reduktion von Aldehyden aus der Roelen'schen Oxosynthese oder der Dimerisierung von ungesättigten Fettsäuren anfallen. Bevorzugt sind technische Fettsäuren mit 12 bis 18 Kohlenstoffatomen wie beispielsweise Kokos-, Palm-, Palmkemoder Talgfettsäure, vorzugsweise in gehärteter bzw. teilgehärteter Form. Es hat sich als besonders vorteilhaft erwiesen, die Carbonsäuren in solchen Mengen einzusetzen, daß im Durchschnitt Diamide resultieren. Aus anwendungstechnischen Gründen besonders bevorzugt sind des weiteren alkoxylierte Fettsäureamidoamine der Formel (IV), in der R⁸CO für einen linearen, gesättigten Acylrest mit 12 bis 18 Kohlenstoffatomen, R⁹, R¹⁰, R¹¹ und R¹² jeweils für Wasserstoff, R¹¹ für R⁸CO, A für eine Ethylengruppe, p und q jeweils für 2, y für 1 und z für Zahlen von 5 bis 10 steht.

Die Alkoxylierung kann in an sich bekannter Weise erfolgen, d.h. Ethylenoxid, Propylenoxid oder deren Gemische werden in Gegenwart saurer, vorzugsweise aber basischer Katalysatoren, wie z.B. Natriummethylat oder calcinierter Hydrotalcit angelagert. Es entstehen nichtionische Verbindungen, die jedoch in saurer Lösung rasch protoniert werden und dann ein pseudokationisches Verhalten zeigen.

Im Sinne der Erfindung können die Detergensgemische die Komponenten (a) und (b) im Gewichtsverhältnis 10 : 90 bis 90 : 10, vorzugsweise 25 : 75 bis 75 : 25 und insbesondere 60 : 40 bis 80 : 20 enthalten.

### Gewerbliche Anwendbarkeit

Die Detergensgemische können zur Herstellung der kosmetischen Zubereitungen in Mengen von 1 bis 50, vorzugsweise 5 bis 35 und insbesondere 10 bis 25 Gew.-% verwendet werden. Abgesehen von der Möglichkeit, die Mischungen unmittelbar für den angegebenen Zweck zu einzusetzen, besteht die einfachste Anwendungsform darin, sie mit Wasser auf die gewünschte Anwendungskonzentration zu verdünnen.

### Tenside

In diesem Zusammenhang ist es möglich, den Zubereitungen auch weitere Zusatzstoffe hinzuzufügen, insbesondere weitere Tenside, die mit den Komponenten (a) und (b) kompatibel sind. Hierbei handelt es sich in erster Linie um weitere nichtionische oder kationische bzw. amphotere oder zwitterionische Tenside. Typische Beispiele für **nichtionische Tenside** sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für **kationische Tenside** sind quartäre Ammoniumverbindungen und monomere Esterquats, insbesondere quaternierte Fettsäuretrialkanolaminestersalze. Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Über sichtsarbeiten beispielsweise **J.Falbe (ed.), "Surfactants in Consumer Products", Springer Verlag, Berlin, 1987, S. 54-124** oder **J.Falbe (ed.), "Katalysatoren, Tenside und Mineralöladditive", Thieme Verlag, Stuttgart, 1978, S. 123-217** verwiesen.

### Hilfs- und Zusatzstoffe

In Abhängigkeit des Einsatzzweckes, also Textil- oder Haaravivage, können die unter Verwendung der Detergensgemische erhältlichen Zubereitungen weitere typische Hilfs- und Zusatzstoffe enthalten, als da sind: Ölkörper, Emulgatoren, Überfettungsmittel, Perlglanzwachse, Stabilisatoren, Konsistenzgeber, Verdickungsmittel, Kationpolymere, Siliconverbindungen, biogene Wirkstoffe, Antischuppenmittel, Filmbildner, Konservierungsmittel, Hydrotrope, Solubilisatoren, UV-Lichtschutzfilter, Insektenrepellentien, Selbstbräuner, Parfümöle, Farbstoffe und dergleichen:

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, pflanzliche Öle, verzweigte primäre Alkohole, substituierte Cyclohexane, lineare C₆-C₂₂-Fettalkoholcarbonate, Guerbetcarbonate, Ester der Benzoesäure mit linearen und/oder verzweigten C₆-C₂₂-Alkoholen (z.B. Finsolv® TN), Dialkylether, Ringöffnungsprodukte von epoxidierten Fettsäureestem mit Polyolen, Siliconöle und/oder aliphatische bzw. naphthenische Kohlenwasserstoffe in Betracht.

Als **Emulgatoren** kommen beispielsweise nichtionogene Tenside aus mindestens einer der folgenden Gruppen in Frage:
(1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) C_{12/18}-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(4) Alkylmono- und -oligoglycoside mit 8 bis 22 Kohlenstoffatomen im Alkylrest und deren ethoxylierte Analoga;
(5) Anlagerungsprodukte von 15 bis 60 Mol Ethylenoxid an Ricinusöl und/oder gehärtetes Ricinusöl;
(6) Polyol- und insbesondere Polyglycerinester, wie z.B. Polyglycerinpolyricinoleat, Polyglycerinpoly-12-hydroxystearat oder Polyglycerindimerat. Ebenfalls geeignet sind Gemische von Verbindungen aus mehreren dieser Substanzklassen;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
(9) Trialkylphosphate sowie Mono-, Di- und/oder Tri-PEG-alkylphosphate;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol gemäß **DE-PS 1165574** und/oder Mischester von Fettsäuren mit 6 bis 22 Kohlenstoffatomen, Methylglucose und Polyolen, vorzugsweise Glycerin sowie
(13) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt.

C_{8/18}-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquaternierte Difettsäuretriethanolaminester-Salze, besonders bevorzugt sind.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Perlglanzwachse** kommen beispielsweise in Frage : Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als **Konsistenzgeber** kommen in erster Linie Fettalkohole mit 12 bis 22 und vorzugsweise 16 bis 18 Kohlenstoffatomen und daneben Partialglyceride in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinyl-imidazol-Polymere, wie z.B. Luviquat® (BASF), Kondensationsprodukte von Polyglycolen und Aminen, quaternierte Kollagenpolypeptide, wie beispielsweise Lauryldimonium hydroxypropyl hydrolyzed collagen (Lamequat®L/Grünau), quaternierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der **FR-A 2252840** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Typische Beispiele für Fette sind Glyceride, als Wachse kommen u.a. Bienenwachs, Camaubawachs, Candelillawachs, Montanwachs, Paraffinwachs oder Mikrowachse gegebenenfalls in Kombination mit hydrophilen Wachsen, z.B. Cetylstearylalkohol oder Partialglyceriden in Frage. Als **Stabilisatoren** können Metallsalze von Fettsäuren, wie z.B. Magnesium-, Aluminium- und/oder Zinkstearat eingesetzt werden. Unter **biogenen Wirkstoffen** sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen. Als **Antischuppenmittel können** Climbazol, Octopirox und Zinkpyrethion eingesetzt werden. Gebräuchliche Filmbildner sind beispielsweise Chitosan, mikrokristallines Chitosan, quaterniertes Chitosan, Polyvinylpyrrolidon, Vinyl-pyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen. Als **Quellmittel** für wäßrige Phasen können Montmorillonite, Clay Mineralstoffe, Pemulen sowie alkylmodifizierte Carbopoltypen (Goodrich) dienen.

Unter **UV-Lichtschutzfiltern** sind organische Substanzen zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
- 3-Benzylidencampher und dessen Derivate, z.B. 3-(4-Methylbenzyliden)campher;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäureisopentylester, 2-Cyano-3-phenyl-zimtsäure-2-ethylhexylester (Octocrylene);
- Ester der Salicylsäure, vorzugsweise Salicylsäure-2-ethylhexylester, Salicylsäure-4-isopropylbenzylester, Salicylsäurehomomenthylester;
- Derivate des Benzophenons, vorzugsweise 2-Hydroxy-4-methoxybenzaphenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyltriazon.
- Propan-1,3-dione, wie z.B. 1-(4-tert.Butylphenyl)-3-(4'methoxyphenyl)propan-1,3-dion;

Als wasserlösliche Substanzen kommen in Frage:
- 2-Phenyibenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bomylidenmethyl)benzolsulfonsäure und 2-Methyl-5-(2-oxo-3-bomyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion oder 1-Phenyl-3-(4'-isopropylphenyl)-propan-1,3-dion. Die UV-A und UV-B-Filter können selbstverständlich auch in Mischungen eingesetzt werden. Neben den genannten löslichen Stoffen kommen für diesen Zweck auch unlösliche Pigmente, nämlich feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk), Bariumsulfat und Zinkstearat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Superoxid-Dismutase, Tocopherole (Vitamin E) und Ascorbinsäure (Vitamin C).

Zur Verbesserung des Fließverhaltens können ferner **Hydrotrope,** wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin.

Als **Konservierungsmittel** eignen sich beispielsweise Phenoxyethanol, Formaldehydlösung, Parabene, Pentandiol oder Sorbinsäure. Als **Insekten-Repellentien** kommen N,N-Diethyl-m-touluamid, 1,2-Pentandiol oder Insect repellent 3535 in Frage, als **Selbstbräuner** eignet sich Dihydroxyaceton.

Als **Parfümöle** seien genannt die Extrakte von Blüten (Lavendel, Rosen, Jasmin, Neroli), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Moschus, Zibet und Castoreum. Als synthetische bzw. halbsynthetische Parfümöle kommen Ambroxan, Eugenol, Isoeugenol, Citronellal, Hydroxycitronellal, Geraniol, Citronellol, Geranylacetat, Citral, Ionon und Methylionon in Betracht.

Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt- oder Heißprozesse erfolgen; vorzugsweise arbeitet man nach der Phaseninversionstemperatur-Methode.

### Beispiele

**Anwendungstechnische Beispiele.** Es wurden sieben Detergensmischungen hergestellt und hinsichtlich Viskosität, Lagerstabilität, Löslichkeit, Dispergierbarkeit, Weichgriff und Naßkämmbarkeit untersucht. Die Viskosität wurde nach Brookfield in einem RVF-Viskosimeter (Spindel 1, 10 Upm) getestet. Die Bestimmung des Weichgriffs erfolgte nach Zwangsapplikation der Testgemische auf Baumwollgewebe durch ein Panel von 6 erfahrenen Testern. Hierbei bedeutet (1) sehr weich, (2) weich, (3) hart und (4) sehr hart. Angegeben sind die Mittelwerte des Panels aus drei Testzyklen. Zur Bestimmung der Trockenkämmbarkeit wurden die statische Aufladung zwischen den Faserfilamenten vor und nach Behandlung mit 1 Gew.-%igen Testlösungen bestimmt, die ein Maß für die Kämmarbeit darstellt. Die Mischungen 1 bis 5 sind erfindungsgemäß, die Mischungen V1 und V2 dienen zum Vergleich. Die Ergebnisse sind in Tabelle 1 zusammengefaßt.

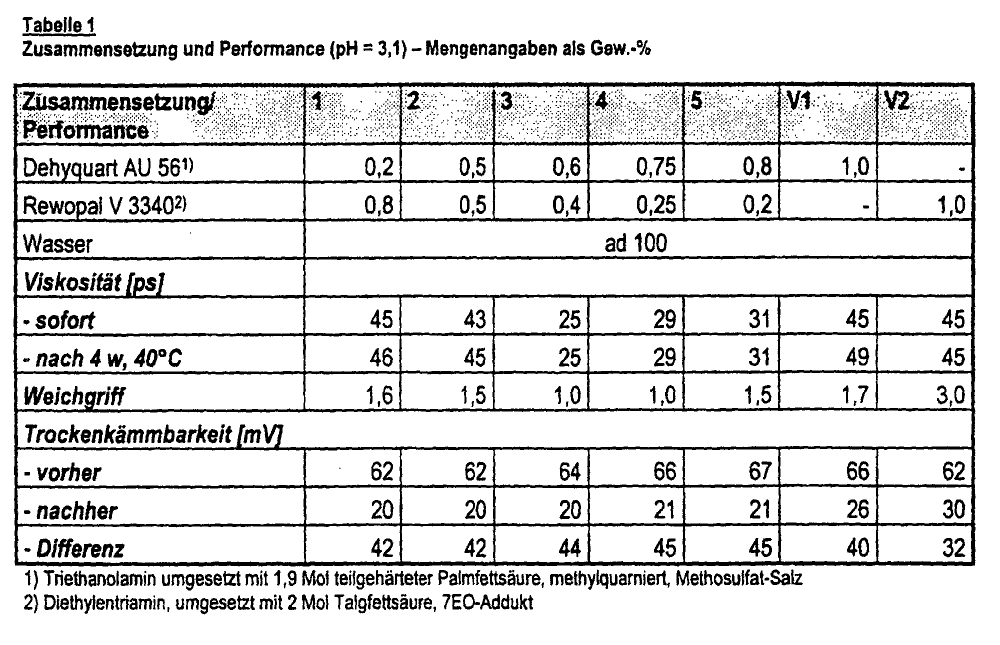

## Patentansprüche

1. Verwendung von Detergensgemischen, enthaltend
(a) Esterquats, erhältlich durch Kondensation von Fettsäuren mit Alkanolaminen und nachfolgende Quaternierung der Alkanolaminoligoester, und
(b) alkoxylierte Fettsäureamidoamine der Formel **(IV)** einsetzt, 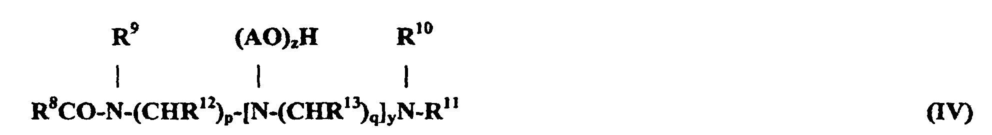 in R⁸CO für einen aliphatischen, linearen oder verzweigten, gesättigten oder ungesättigten Acylrest mit 6 bis 22 Kohlenstoffatomen, R⁹ und R¹⁰ unabhängig voneinander für Wasserstoff oder einen gegebenenfalls hydroxysubstituierten Alkylrest mit 1 bis 4 Kohlenstoffatomen, R¹¹ für Wasserstoff, einen Alkylrest mit 1 bis 4 Kohlenstoffatomen oder R⁸CO, R¹² und R¹³ unabhängig voneinander für Wasserstoff oder eine Methylgruppe, A für eine lineare oder verzweigte Alkylen-gruppe mit 2 bis 4 Kohlenstoffatomen, p und q unabhängig voneinander für Zahlen von 1 bis 3, y für Zahlen von 1 bis 3 und z für Zahlen von 1 bis 20 steht,
zur Herstellung von kosmetischen Zubereitungen.

2. Verwendung nach Anspruch 1, **dadurch gekennzeichnet, daß** man Esterquats der Formel **(I)** einsetzt, 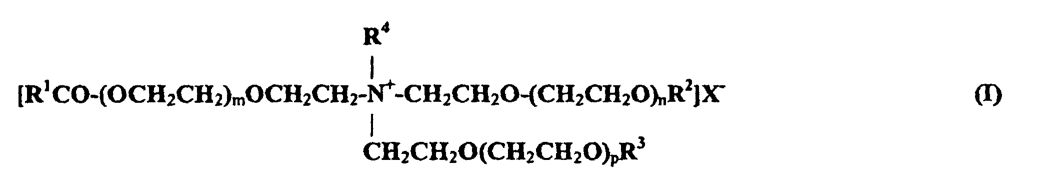 in der R¹CO für einen Acylrest mit 6 bis 22 Kohlenstoffatomen, R² und R³ unabhängig voneinander für Wasserstoff oder R¹CO, R⁴ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht.

3. Verwendung nach Anspruch 1, **dadurch gekennzeichnet, daß** man Esterquats der Formel **(II)** einsetzt, 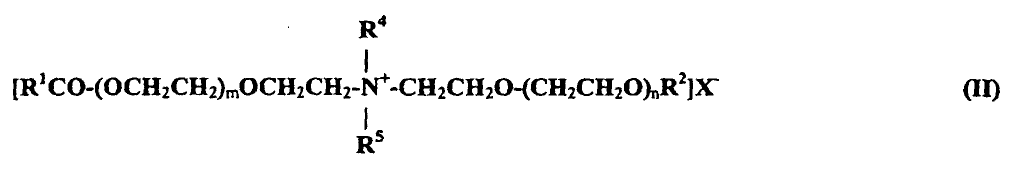 in der R¹CO für einen Acylrest mit 6 bis 22 Kohlenstoffatomen, R² für Wasserstoff oder R¹CO, R⁴ und R⁵ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

4. Verwendung nach Anspruch 1, **dadurch gekennzeichnet, daß** man Esterquats der Formel **(III)** einsetzt, 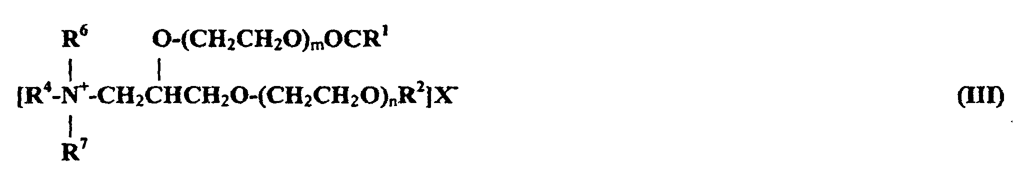 in der R¹CO für einen Acylrest mit 6 bis 22 Kohlenstoffatomen, R² für Wasserstoff oder R¹CO, R⁴, R⁶ und R⁷ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

5. Verwendung wenigstens einem der Ansprüche 1 bis 4, **dadurch gekennzeichnet, daß** man als Komponente (b) Fettsäureamidoamine der Formel **(IV)** einsetzt, in der R⁸CO für einen linearen, gesättigten Acylrest mit 12 bis 18 Kohlenstoffatomen, R⁹, R¹⁰, R¹² und R¹³ jeweils für Wasserstoff, R¹¹ für R⁸CO, A für eine Ethylengruppe, p und q jeweils für 2, y für 1 und z für Zahlen von 5 bis 10 steht.

6. Verwendung nach den Ansprüchen 1 bis 5, **dadurch gekennzeichnet, daß** man die Komponenten (a) und (b) im Gewichtsverhältnis 90 : 10 bis 10 : 90 einsetzt.

7. Verwendung nach Anspruch 6, **dadurch gekennzeichnet, daß** man die Komponenten (a) und (b) im Gewichtsverhältnis 60 : 40 bis 80 : 20 einsetzt.

8. Verwendung der Detergensgemische nach den Ansprüchen 1 bis 7 zu Herstellung von Haarpflegemitteln.

9. Verwendung der Detergensgemische nach den Ansprüchen 1 bis 7 zu Herstellung von Haarkonditioniermitteln.

## Claims

1. The use of detergent mixtures containing
(a) esterquats obtainable by condensation of fatty acids with alkanolamines and subsequent quaternization of the alkanolamine oligoesters and
(b) alkoxylated fatty acid amidoamines corresponding to formula **(IV)**: 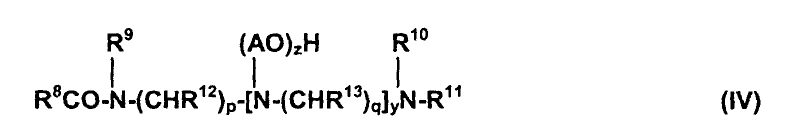
in which R⁸CO is an aliphatic, linear or branched, saturated or unsaturated C₆₋₂₂ acyl group, R⁹ and R¹⁰ independently of one another represent hydrogen or an optionally hydroxysubstituted alkyl group containing 1 to 4 carbon atoms, R¹¹ is hydrogen or a C₁₋₄ alkyl group or has the same meaning as R⁸CO, R¹² and R¹³ independently of one another represent hydrogen or a methyl group, A is a linear or branched C₂₋₄ alkylene group, p and q independently of one another are numbers of 1 to 3, y is a number of 1 to 3 and z is a number of 1 to 20,
for the production of cosmetic preparations.

2. The use claimed in claim 1, **characterized in that** esterquats corresponding to formula **(I)**: 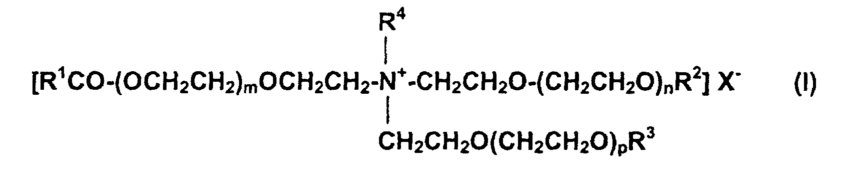 in which R¹CO is an acyl group containing 6 to 22 carbon atoms, R² and R³ independently of one another represent hydrogen or have the same meaning as R¹CO, R⁴ is an alkyl group containing 1 to 4 carbon atoms or a (CH₂CH₂O)_{q}H group, m, n and p together stand for 0 or numbers of 1 to 12, q is a number of 1 to 12 and X is halide, alkyl sulfate or alkyl phosphate, are used.

3. The use claimed in claim 1, **characterized in that** esterquats corresponding to formula **(II)**: 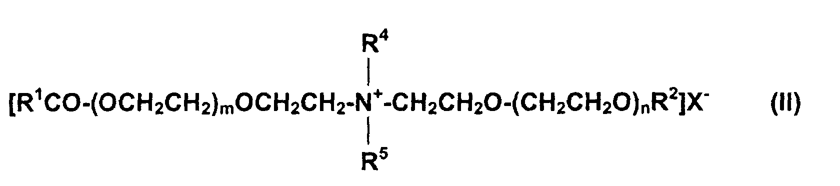 in which R¹CO is an acyl group containing 6 to 22 carbon atoms, R² is hydrogen or has the same meaning as R¹CO, R⁴ and R⁵ independently of one another are alkyl groups containing 1 to 4 carbon atoms, m and n together stand for 0 or numbers of 1 to 12 and X stands for halide, alkyl sulfate or alkyl phosphate,
are used.

4. The use claimed in claim 1, **characterized in that** esterquats corresponding to formula **(III)**: 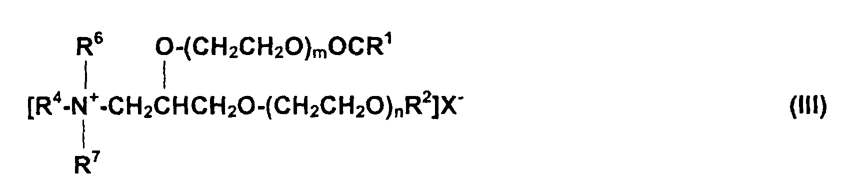 in which R¹CO is an acyl group containing 6 to 22 carbon atoms, R² is hydrogen or has the same meaning as R¹CO, R⁴, R⁶ and R⁷ independently of one another are alkyl groups containing 1 to 4 carbon atoms, m and n together stand for 0 or numbers of 1 to 12 and X stands for halide, alkyl sulfate or alkyl phosphate,
are used.

5. The use claimed in at least one of claims 1 to 4, **characterized in that** fatty acid amidoamines of formula **(IV),** in which R⁸CO is a linear saturated acyl group containing 12 to 18 carbon atoms, R⁹, R¹⁰, R¹² and R¹³ are each hydrogen, R¹¹ has the same meaning as R⁸CO, A is an ethylene group, p and q are each 2, y is 1 and z is a number of 5 to 10, are used as component (b).

6. The use claimed in claims 1 to 5, **characterized in that** components (a) and (b) are used in a ratio by weight of 90:10to 10:90.

7. The use claimed in claim 6, **characterized in that** components (a) and (b) are used in a ratio by weight of 60:40 to 80:20.

8. The use of the detergent mixtures as claimed in claims 1 to 7 for the production of hair care preparations.

9. The use of the detergent mixtures claimed in claims 1 to 7 for the production of hair conditioners.

## Revendications

1. Utilisation de mélanges détergents contenant :
(a) des esters d'ammonium quaternaire que l'on peut obtenir par condensation d'acides gras avec des alcanolamines puis par quaternisation des oligoesters d'alcanolamines et
(b) des amidoamines d'acides gras alcoxylées de formule (IV), 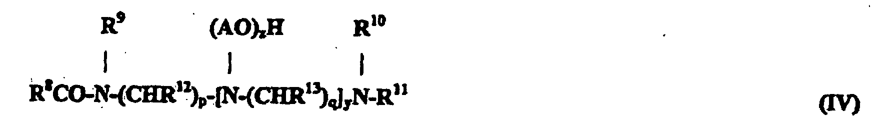
dans laquelle R⁸CO représente un radical acyle aliphatique, linéaire ou ramifié, saturé ou insaturé, comportant de 6 à 22 atomes de carbone, R⁹ et R¹⁰ représentent indépendamment l'un de l'autre l'hydrogène ou un radical alkyle éventuellement hydroxy-substitué comportant de 1 à 4 atomes de carbone, R¹¹ représente l'hydrogène, un radical alkyle comportant de 1 à 4 atomes de carbone ou R⁸CO, R¹² et R¹³ représentent indépendamment l'un de l'autre l'hydrogène ou un groupe méthyle, A représente un groupe alkylène linéaire ou ramifié comportant de 2 à 4 atomes de carbone, p et q représentent indépendamment l'un de l'autre des nombres allant de 1 à 3, y représente des nombres allant de 1 à 3, et z représente des nombres allant de 1 à 20,
pour la fabrication de préparations cosmétiques.

2. Utilisation selon la revendication 1,
**caractérisée en ce qu'**
on utilise des esters d'ammonium quaternaire de formule (I) 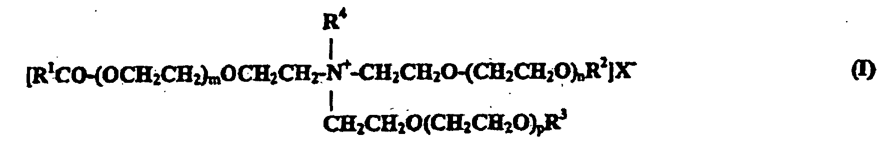 dans laquelle R¹CO représente un radical acyle comportant de 6 à 22 atomes de carbone, R² et R³ représentent indépendamment l'un de l'autre l'hydrogène ou R¹CO, R⁴ représente un radical alkyle comportant de 1 à 4 atomes de carbone, ou un groupe (CH₂CH₂O)_{q}H, m, n et p valent au total 0 ou des nombres allant de 1 à 12, q représente des nombres allant de 1 à 12, et X représente un halogénure, un sulfate d'alkyle ou un phosphate d'alkyle.

3. Utilisation selon la revendication 1,
**caractérisée en ce qu'**
on utilise des esters d'ammonium quaternaire de formule (II) 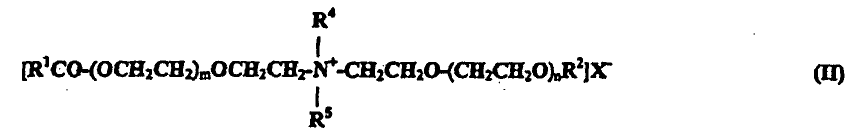 dans laquelle R¹CO représente un radical acyle comportant de 6 à 22 atomes de carbone, R² représente l'hydrogène ou R¹CO, R⁴ et R⁵ représentent indépendamment l'un de l'autre des radicaux alkyle comportant de 1 à 4 atomes de carbone, m et n valent au total 0 ou des nombres allant de 1 à 12, et X représente un halogénure, un sulfate d'alkyle ou un phosphate d'alkyle.

4. Utilisation selon la revendication (I),
**caractérisée en ce qu'**
on utilise des esters d'ammonium quaternaire de formule (III) 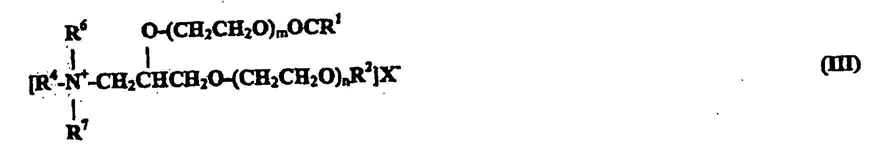 dans laquelle R¹CO représente un radical acyle comportant de 6 à 22 atomes de carbone, R² représente un hydrogène ou R¹CO, R⁴, R⁶ et R⁷ représentent indépendamment les uns des autres des radicaux alkyle comportant 1 à 4 atomes de carbone, m et n valent au total 0 ou des nombres allant de 1 à 12, et X représente un halogénure, un sulfate d'alkyle ou un phosphate d'alkyle.

5. Utilisation selon au moins l'une des revendications 1 à 4,
**caractérisée en ce qu'**
on utilise comme composant (b) des amidoamines d'acides gras de formule (IV), dans laquelle R⁸CO représente un radical acyle linéaire saturé comportant de 12 à 18 atomes de carbone, R⁹, R¹⁰, R¹² et R¹³ représentent chacun l'hydrogène, R¹¹ représente R⁸CO, A représente un groupe éthylène, p et q valent chacun 2, y vaut 1 et z représente des nombres allant de 5 à 10.

6. Utilisation selon les revendications 1 à 5,
**caractérisée en ce qu'**
on utilise les composants (a) et (b) dans un rapport pondéral de 90 : 10 à 10 :90.

7. Utilisation selon la revendication 6,
**caractérisée en ce qu'**
on utilise les composants (a) et (b) dans un rapport pondéral de 60 :40 à 80 :20.

8. Utilisation des mélanges détergents selon les revendications 1 à 7 pour la fabrication de produits de soins capillaires.

9. Utilisation des mélanges détergents selon les revendications 1 à 7, pour la fabrication de produits de conditionnement des cheveux.
